# EUROPÄISCHE PATENTSCHRIFT

(11) **EP 3 758 768 B1**
(45) Veröffentlichungstag und Bekanntmachung des Hinweises auf die Patenterteilung: **15.04.2026**
(21) Anmeldenummer: 19711504.1
(22) Anmeldetag: 26.02.2019
(51) Int. Cl.: A61M 1/16, A61M 1/36

(54) **STEUER- ODER REGELVORRICHTUNG FÜR EINE HERZ-LUNGEN-MASCHINE**
CONTROL OR REGULATION DEVICE FOR A HEART-LUNG MACHINE
DISPOSITIF DE COMMANDE OU DE RÉGULATION POUR UNE MACHINE CARDIO-PULMONAIRE

(30) Priorität: 26.02.2018 DE 102018001467; 03.07.2018 LU 100849
(43) Veröffentlichungstag der Anmeldung: 06.01.2021
(73) Patentinhaber: Hemovent GmbH, 52076 Aachen (DE)
(72) Erfinder: MARSEILLE, Oliver, 52066 Aachen (DE); KORTHAUER, Jürgen, 46519 Alpen (DE); WEBER, Markus, Spring Valley, California 91977 (US); NÖTZEL, Stefan, 52064 Achen (DE)
(74) Vertreter: Sattler de Sousa e Brito, Clara
(86) Internationale Anmeldenummer: PCT/EP2019/054723
(87) Internationale Veröffentlichungsnummer: WO 2019/162525

(56) Entgegenhaltungen:
- WO-A1-2016/100512
- US-A- 4 828 543
- US-A- 5 810 759
- US-A1- 2005 004 480
- US-A1- 2017 252 505
- US-A1- 2017 368 247

## Beschreibung

Die Erfindung betrifft eine Herz-Lungen-Maschine mit einer Steuer- oder Regelvorrichtung.

Patienten mit Herzversagen oder Lungenversagen können durch eine extrakorporal eingesetzte Herz-Lungen-Unterstützung vor einer weiteren Verschlechterung des Zustandes bewahrt bzw. am Leben erhalten werden. Herz bzw. Lunge werden zum einen entlastet, um sie einer Therapie zugänglich zu machen, während gleichzeitig deren Organfunktion durch externe Unterstützung aufrechterhalten wird. Hierbei wird der Patient durch geeigneten Kanülen mit einem extrakorporalen Kreislauf verbunden, der die Herz- und Lungenfunktion gewährleistet.

Eine Herz-Lungen-Maschine besteht aus einer Blutpumpe und einer künstlichen Lunge. Das Blut wird durch einen peripheren oder zentralen Zugang mit Kanülen aus dem venösen Gefäßsystem entnommen und nach Anreicherung mit Sauerstoff auf der venösen oder arteriellen Seite wieder zugefügt. So werden die lebenswichtigen Organe mit frischem Blut versorgt, während der Patient transportiert oder einer weiteren Therapie zugeführt werden kann. Die Blutpumpe ist in der Regel mit einer Antriebskonsole fest verbunden und durch eine Magnetkupplung wird die Antriebsenergie von einem Pumpenmotor auf einen Rotor der Blutpumpe übertragen. Auch werden Rollerpumpen eingesetzt, bei denen ein Pumpenschlauch in die Antriebskonsole eingelegt wird. Die künstliche Lunge, auch Oxygenator oder Gastauscher genannt, befindet sich in Flussrichtung hinter der Blutpumpe.

Die üblichen Kontrolleinheiten für die Blutpumpe überwachen die Pumpfunktion des Systems hinsichtlich der Blutfördermenge, der Blutdrücke und geben ggf. Alarme. Der Blutfluss wird durch den Anwender an der Kontrolleinheit durch die Pumpendrehzahl eingestellt.

Die Gasversorgung des Oxygenators wird durch einen von der Kontrolleinheit separaten Gasblender gewährleistet, der mit Sauerstoff und Druckluft versorgt wird. Je nach Bedarf kann die Gasflussmenge und Zusammensetzung gewählt werden. Teilweise wird der Oxygenator auch nur mit Sauerstoff mit gewünschtem Volumenstrom durch eine Drosseleinrichtung versorgt.

Die WO 2016/100512 A1 offenbart eine Vorrichtung umfassend eine Blutpumpe und einen Oxygenator, welche bevorzugt mit elektrischer Energie beispielsweise aus Batterien versorgt wird.

Die US 5,810,759 offenbart eine Vorrichtung mit einer Blutpumpe und einem Oxygenator, wobei die Steuerung der Vorrichtung nur vielen Messdaten erfolgen kann, was die Vorrichtung hochkomplex macht.

Ein Nachteil bei den bekannten Herz-Lungen-Maschinen besteht darin, dass die Bedienung der Gasversorgung, die für die Therapie von gleicher Bedeutung wie die Regelung des Blutflusses ist, räumlich getrennt angeordnet ist und damit erschwert wird und unübersichtlich ist. Dadurch wird die Patientensicherheit und Therapiequalität beeinflusst. Beim klinisch üblichen Einsatz einer Sauerstoffquelle mit Drosseleinrichtung existiert darüber hinaus keine Alarm- oder Hinweisfunktion zur Funktion der Gasquelle. Ein weiterer Nachteil besteht darin, dass Herz-Lungen-Maschinen aus einer Vielzahl von Bestandteilen bestehen. Dadurch erschwert sich der Transport der Herz-Lungen-Maschine und deren Bedienung.

Eine Aufgabe der Erfindung besteht daher darin, die Sicherheit und Therapiequalität zu verbessern, sowie den Transport der Herz-Lungen-Maschine zu vereinfachen.

Die Aufgabe wird durch eine Herz-Lungen-Maschine mit einer Steuer- oder Regelvorrichtung nach dem Anspruch 1 gelöst. Die Gegenstände der Unteransprüche geben bevorzugte Ausführungsformen wieder.

Die erfindungsgemäße Herz-Lungen-Maschine mit einer Steuer- oder Regelvorrichtung weist den Vorteil auf, dass durch ein einziges Gerät sowohl der Blutvolumenstrom als auch der Volumenstrom des durch die Gasaustauscheinheit strömenden Gases eingestellt werden kann. Dadurch erhöht sich die Bediensicherheit, die Therapieüberwachung, und der Transport und die Bedienung der Herz-Lungen-Maschine vereinfachen sich. Insbesondere müssen nicht mehr zwei voneinander separate Geräte bewegt werden, sondern lediglich ein einziges Gerät, nämlich die Steuer- oder Regelvorrichtung. Ein weiterer Vorteil der erfindungsgemäßen Herz-Lungen-Maschine mit der Steuer- oder Regelvorrichtung besteht darin, dass die wesentlichen Bedienungsschritte an einem Gerät erfolgen, wodurch Bedienungsfehler, insbesondere bei der Inbetriebnahme, vermieden werden.

Die Steuer und Regelvorrichtung besteht aus einem kompakten Gerät insbesondere mit einem Gehäuse, an das direkt die blutführenden Einmalkomponenten Pumpe und Gastauscher angeschlossen und mit Energie bzw. Gas versorgt werden. Weitere Anschlüsse oder Zusatzgeräte werden nicht benötigt. Die Versorgung des Geräts erfolgt nur durch Gas- und ggf. Stromnetzanschluss. Die Pumpenregelung und Gasdosierung sind in das Gerät integriert. Daher wird keine separate Pumpenkonsole oder Gasflusskontrolle benötigt. Das Gerät ist als tragbares Gerät ausgeführt.

In einer Variante des Geräts kann für den Antrieb der Blutpumpe der wiederverwendbare Aktuator wie beispielsweise ein Elektromotor auch außerhalb des Gehäuses angeordnet sein und beispielsweise durch ein Kabel mit diesem verbunden sein.

In einer Ausführung kann das Gerät ein Gehäusevolumen von weniger als 2 Liter und ein Gewicht von weniger als 2 kg haben. Durch die kompakte und leichte Bauform ist die Variante des Geräts besonders gut für einen Transport von Patienten geeignet. Auch kann es aufgrund der kompakten Bauform sehr nah am Patienten positioniert werden.

Das Gas kann aus einem Einzelgas bestehen. Alternativ kann das Gas ein Gasgemisch sein, das aus mehreren Einzelgasen und/oder einem Einzelgas und einem oder mehreren weiteren Gas besteht.

Bei einer besonderen Ausführung, kann die Steuer- oder Regeleinheit derart ausgebildet und dazu bestimmt sein, zum Steuern oder Regeln des Blutvolumenstroms die nicht zur Steuer- oder Regelvorrichtung gehörende Blutpumpe elektrisch und/oder pneumatisch anzutreiben. Dabei kann die Blutpumpe als eine Kreiselpumpe ausgeführt sein, die elektrisch über eine Drehzahlsteuerung oder pneumatisch über eine Gasversorgungssteuerung gesteuert oder geregelt wird. Der durch die Blutpumpe geförderte Blutvolumenstrom hängt von der Drehzahlsteuerung und/oder der Gasversorgungssteuerung ab. Die Blutpumpe kann eine Pumpe mit saugendem oder pulsierendem Strom sein.

Nach einem weiteren unabhängig erfinderischen Aspekt, kann die Steuer- oder Regeleinheit den durch die nicht zur Steuer- oder Regelvorrichtung gehörende Gasaustauscheinheit strömenden Gasvolumenstrom abhängig oder unabhängig von dem Blutvolumenstrom, insbesondere automatisch, steuern oder regeln. Insbesondere kann die Steuer- oder Regeleinheit den Gasvolumenstrom insbesondere abhängig, von einem voreingestellten Blutvolumenstrom steuern oder regeln. Der Blutvolumenstrom kann somit zeitlich vor der Steuerung oder Regelung des Gasvolumenstroms durch den Benutzer, beispielsweise durch Betätigen eines Bedienknopfs, eingestellt werden. In Falle der automatischen Steuerung in Abhängigkeit vom Blutstrom, muss insbesondere keine manuelle Anpassung des Gasstromes mehr erfolgen.

Dabei kann die Steuer- oder Regeleinheit den Gasvolumenstrom und/oder den Blutvolumenstrom derart steuern oder regeln, dass ein vorgebbares Verhältnis zwischen dem Gasvolumenstrom und dem Blutvolumenstrom eingestellt werden kann. Dazu kann die Steuer- oder Regeleinheit wenigstens eine Eingabemöglichkeit aufweisen, mittels der ein Anwender das gewünschte Verhältnis eingeben kann. Alternativ oder zusätzlich kann die Steuer- oder Regeleinheit den Blutvolumenstrom und/oder den Gasvolumenstrom derart steuern oder regeln, dass ein zeitlich veränderbares oder durch Messparameter beeinflussbares Verhältnis zwischen dem Gasvolumenstrom und dem Blutvolumenstrom einstellbar ist.

Bei einer Veränderung des Blutvolumenstroms kann die Steuer- oder Regeleinheit den Gasvolumenstrom automatisch auf einen dem Blutvolumenstrom zugeordneten Wert steuern oder regeln. Somit ist es nicht notwendig, dass der Anwender, den Blutvolumenstrom kontinuierlich überwacht und bei Änderungen des Blutvolumenstroms eingreifen muss. Insbesondere kann der Anwender das gewünschte Verhältnis zwischen dem Gasvolumenstrom und dem Blutvolumenstrom einmalig eingeben und die Steuer-oder Regelvorrichtung wird den Gasvolumenstrom selbsttätig an einen geänderten Blutvolumenstrom anpassen. Da nur eine einzige Steuer- oder Regelvorrichtung den Gasvolumenstrom und den Blutvolumenstrom steuert oder regelt, muss der Anwender das gewünschte Verhältnis nur in einem einzigen Gerät eingeben, was die Anwendung vereinfacht. Darüber hinaus kann beim Einschalten der Blutpumpe und einem damit verbundenen Blutvolumenstrom die Steuer- oder Regeleinheit gleichzeitig den Gasvolumenstrom auf einen Wert steuern oder regeln, der dem durch die Blutpumpe geförderten Blutvolumenstrom zugeordnet ist.

Außerdem kann wenigstens ein Algorithmus hinterlegt sein, der das Verhältnis von Gasvolumenstrom und Blutvolumenstrom steuert oder regelt. Die Steuer und Regeleinheit paßt nach Vorgabe bzw. Änderung des Blutvolumenstroms durch den Anwender den Gasvolumenstrom zum Gasaustauscher an. Das Gerät benötigt hierzu keine externen Sensoren z.B. zur Blutgasüberwachung sondern nutzt hierzu die vom Anwender eingestellte Blutflussrate.

Die Regelung kann ohne elektronische Komponenten erfolgen. Der Gasfluss durch den Gastauscher kann durch mechanische oder pneumatische Kopplung in Abhängigkeit zum Blutflusseinstellmechanismus (Stellknopf für Blutfluss) geregelt werden.

Die Steuerung oder Regelung kann auch abhängig von der Zeit oder anderen Messparametern erfolgen. Wenigstens ein Algorithmus kann in einer elektrischen Speichereinheit der Steuer- oder Regelvorrichtung gespeichert sein.

Die Gasaustauscheinheit kann ein Oxygenator sein. Ein Oxygenator ist ein Gerät, bei dem Blut mit Sauerstoff angereichert und Kohlenstoffdioxid aus dem Blut entfernt werden kann. Somit kann mittels des Oxygenators sowohl kurzfristig als auch über längere Zeiträume hinweg die Lunge ersetzt oder unterstützt werden.

Bei einer besonderen Ausführung kann Steuer- oder Regeleinheit eine Mischeinrichtung aufweisen. In der Mischeinrichtung können wenigstens zwei Gase miteinander vermischt werden. Insbesondere kann in der Mischeinrichtung Sauerstoff mit Luft, insbesondere Druckluft, vermischt werden. Darüber hinaus ist es in einzelnen Fällen möglich, dass in der Mischeinrichtung ein Vermischen von Sauerstoff mit Luft, wie Druckluft, und/oder einem weiteren Gas, wie beispielsweise CO₂ (Kohlendioxid) und/oder NO (Stickstoffmonoxid) erfolgt. Das aus der Mischeinrichtung strömende Gas kann der Gasaustauscheinheit zugeführt werden.

Die Steuer- oder Regeleinheit kann außerdem eine Einstelleinrichtung aufweisen, mittels der ein Volumenstrom und/oder eine Zusammensetzung des aus der Mischeinrichtung strömenden Gases einstellbar ist. So kann der Anwender mittels der Einstelleinrichtung auf einfache Weise den gewünschten Volumenstrom und/oder die Zusammensetzung des Gases einstellen, das aus der Mischeinrichtung ausströmt. Die Einstelleinrichtung kann mehrere Bedienknöpfe aufweisen, mittels denen der Volumenstrom und/oder die Zusammensetzung des Gases, insbesondere separat, einstellbar ist. Alternativ kann die Einstelleinrichtung ein Touchdisplay aufweisen, mittels dem der Anwender die gewünschten Eingaben tätigen kann. Im Ergebnis kann durch die Mischeinrichtung und die Einstelleinrichtung auf einfache Weise der Volumenstrom und die Zusammensetzung des Gases eingestellt werden, das von der Mischeinrichtung zu der Gasaustauscheinrichtung strömt. Darüber hinaus kann durch die Einstelleinrichtung der der Gasaustauscheinheit zugeführte Gasvolumenstrom auf einfache Weise gesteuert oder geregelt werden.

Die der Mischeinrichtung zugeführten Gase können aus mehreren unterschiedlichen nicht zur Steuer- oder Regelvorrichtung gehörenden Gasquellen stammen. Die Gasquellen sind mit der Steuer- oder Regelvorrichtung fluidisch verbunden. Dabei wird der Steuer- oder Regelvorrichtung durch eine erste Quelle Sauerstoff zugeführt. Durch eine zweite Quelle wird der Steuer- oder Regelvorrichtung Luft, insbesondere Druckluft, zugeführt. Darüber hinaus kann eine dritte Quelle vorhanden sein, mittels der der Steuer-oder Regelvorrichtung ebenfalls Sauerstoff zugeführt werden kann. Die dritte Quelle dient als Notreserve für den Fall, dass die erste Quelle ausfällt. Über weitere Quellen können zusätzliche Mischgase zugeführt werden.

Darüber hinaus kann die Steuer- oder Regeleinheit eine weitere Mischeinrichtung zum Vermischen von Gas und Umgebungsluft aufweisen. Insbesondere kann in der weiteren Mischeinrichtung Sauerstoff mit Umgebungsluft vermischt werden. Zum Ansaugen der Umgebungsluft kann die weitere Mischeinrichtung ein Venturielement aufweisen. Das aus der weiteren Mischeinrichtung strömende Gas kann der Gasaustauscheinheit zugeführt werden.

Die Steuer- oder Regeleinheit kann eine weitere Einstelleinrichtung aufweisen, mittels der ein Volumenstrom und/oder eine Zusammensetzung des aus der weiteren Mischeinrichtung strömenden Gases einstellbar ist. Die weitere Einstelleinrichtung kann mehrere Bedienknöpfe aufweisen, mittels denen der Volumenstrom und/oder die Zusammensetzung des Gases einstellbar ist. Alternativ kann die Einstelleinrichtung ein Touchdisplay aufweisen, mittels dem der Anwender die gewünschten Eingaben tätigen kann. Im Ergebnis kann durch die weitere Mischeinrichtung und die weitere Einstelleinrichtung auf einfache Weise der Volumenstrom und die Zusammensetzung des Gases eingestellt werden, das von der weiteren Mischeinrichtung zu der Gasaustauscheinheit strömt. Darüber hinaus kann durch die weitere Einstelleinrichtung der der Gasaustauscheinheit zugeführte Gasvolumenstrom auf einfache Weise gesteuert oder geregelt werden.

Die Steuer- oder Regeleinheit weist eine andere Einstelleinrichtung auf, mittels der ein Volumenstrom des zur Blutpumpe strömenden Gases steuerbar oder regelbar ist. Dies bietet sich bei Ausführungen an, bei denen die Blutpumpe pneumatisch angesteuert wird. Die Blutpumpe wird durch das Gas derart angesteuert, dass eine Pumpwirkung bewirkt und somit der Blutvolumenstrom gefördert wird. Mittels der Einstelleinrichtung wird somit der Blutvolumenstrom auf einfache Weise gesteuert oder geregelt.

Die andere Einstelleinrichtung kann gasstromaufwärts der Blutpumpe angeordnet sein. Der Blutpumpe kann ausschließlich Sauerstoff zugeführt werden. Es erfolgt somit kein Vermischen des der Blutpumpe zugeführten Gases mit einem anderen Gas, bevor es der Blutpumpe zugeführt wird. Insofern wird auch keine Einstelleinrichtung benötigt, mittels der die Zusammensetzung des zur Blutpumpe strömenden Gases eingestellt werden kann.

Die Steuer oder Regeleinheit weist eine Schalteinrichtung auf, die mit der Blutpumpe fluidisch verbunden und mit der Gasaustauscheinheit fluidisch verbindbar ist. Die Schalteinrichtung kann gasstromabwärts der Blutpumpe und/oder gasstromaufwärts der Gasaustauscheinheit angeordnet sein. Zwei Bauteile sind miteinander fluidisch verbunden, wenn ein Gas und/oder eine Flüssigkeit von einem Bauteil in das andere Bauteil oder umgekehrt strömen kann. Mittels der Schalteinrichtung kann eingestellt werden, ob das aus der Blutpumpe strömende Gas wahlweise der Gasaustauscheinheit zugeführt wird oder an die Umgebung abgegeben wird. Als Umgebung wird auch ein Hohlraum eines Gehäuses der Steuer- oder Regeleinrichtung verstanden.

Bei einer Schaltstellung der Schalteinrichtung kann die Blutpumpe mittels der Schalteinrichtung mit der Gasaustauscheinheit fluidisch verbunden sein. Somit kann das aus der Blutpumpe ausströmende Gas die Schalteinrichtung durchströmen und weiter in Richtung zur Gasaustauscheinheit strömen. Bei einer anderen Schaltstellung der Schalteinrichtung kann die Blutpumpe mit der Umgebung fluidisch verbunden sein. Dies bedeutet, dass bei der anderen Schaltstellung, dass in die Schalteinrichtung eingeströmte Gas vollständig an die Umgebung abgegeben wird.

Die Steuer- oder Regeleinheit kann eine Schalteinstelleinrichtung aufweisen. Die Schalteinstelleinrichtung dient zum Steuern oder Regeln eines Volumenstroms des von der Schalteinrichtung zu der Gasaustauscheinheit zuführbaren Gases. Somit kann auf einfache Weise der Volumenstrom eingestellt werden, der der Gasaustauscheinheit zugeführt wird. Dies ist insbesondere notwendig, um ein respiratorische Alkalose des Patienten zu vermeiden, die auftreten kann, wenn in der Gasaustauscheinheit zu viel Kohlendioxid entfernt wird. Dabei kann die Schalteinstelleinrichtung derart ausgebildet sein, dass der nicht zu der Gasaustauscheinheit zugeführte Teil des Gases an die Umgebung abgegeben wird.

Bei einer besonderen Ausführung kann die Steuer oder Regeleinheit eine Auswahleinrichtung aufweisen, mittels der wahlweise unterschiedliche Betriebsmodi der Steuer- oder Regelvorrichtung einstellbar sind. So kann mittels der Auswahleinrichtung ein erster Betriebsmodus eingestellt werden, bei dem sich die Schalteinrichtung in der Schaltstellung befindet, so dass dasselbe Gas sowohl der Blutpumpe als auch der Gasaustauscheinheit zuführbar ist. Insbesondere kann bei diesem Betriebsmodus sowohl der Blutpumpe als auch der Gasaustauscheinheit reiner Sauerstoff zugeführt werden. Der erste Betriebsmodus eignet sich für Einzelfälle, bei denen das Blut in der Gasaustauscheinheit mit viel Sauerstoff angereichert werden soll. Das der Gasaustauscheinheit zugeführte Gas kann nach der Anreicherung des Blutes mit Sauerstoff an die Umgebung abgegeben werden.

Mittels der Auswahleinrichtung kann ein zweiter Betriebsmodus eingestellt werden, bei dem sich die Schalteinrichtung in der anderen Schaltstellung befindet und bei dem das aus der Mischeinrichtung strömende Gas der Gasaustauscheinheit zuführbar ist. Das aus der Blutpumpe zu der Schalteinrichtung zugeführte Gas wird bei diesem Betriebsmodus vollständig an die Umgebung abgegeben. Bei dem zweiten Betriebsmodus besteht der Vorteil darin, dass die Konzentration des Sauerstoffes in dem der Gasaustauscheinheit zugeführten Gas in der Mischeinrichtung eingestellt werden kann. Somit kann auf einfache Weise verhindert werden, dass das Blut in der Gasaustauscheinheit mit zu viel Sauerstoff angereichert wird. Bei dem zweiten Betriebsmodus werden der Blutpumpe und der Gasaustauscheinheit unterschiedliche Gase zugeführt. So wird der Blutpumpe ein Gas, wie beispielsweise ausschließlich Sauerstoff oder Druckluft, zugeführt, während der Gasaustauscheinheit das aus der Mischeinrichtung ausströmende Gas, wie beispielsweise ein Gemisch aus Sauerstoff und Druckluft, zugeführt wird.

Darüber hinaus kann mittels der Auswahleinrichtung ein dritter Betriebsmodus eingestellt werden, bei dem sich die Schalteinrichtung in der anderen Schaltstellung befindet und bei dem das aus der weiteren Mischvorrichtung strömende Gas der Gasaustauscheinheit zuführbar ist. Dabei kann der Gasaustauscheinheit ein Gasgemisch, das aus Sauerstoff und Umgebungsluft besteht, zugeführt werden. Somit werden auch bei dem dritten Betriebsmodus analog zu dem zweiten Betriebsmodus der Blutpumpe und der Gasaustauscheinheit unterschiedliche Gase zugeführt. Der dritte Betriebsmodus ist bei Einsatzfällen von Vorteil, bei denen dem Anwender keine Druckluft zur Verfügung steht. Dies ist oftmals der Fall, wenn die Herz-Lungen-Maschine außerhalb des Krankenhauses eingesetzt wird.

Bei einer besonderen Ausführung kann die Steuer- und Regelvorrichtung eine Sicherheitseinrichtung aufweisen. Die Sicherheitseinrichtung kann zum Überwachen des Betriebs der Steuer- und Regelvorrichtung dienen. So können durch geeignete Sensoren oder pneumatische-elektrische Schalter die Abweichungen vom Normalbetrieb erfasst werden und/oder mittels der Sicherheitseinrichtung Alarme abgegeben werden. Die Sicherheitseinrichtung kann über Standard oder wiederaufladbare Batterien versorgt werden und so netzunabhängig sein.

Die Alarme können akustisch und/oder optisch den Anwender informieren. Alarme können gegeben werden, wenn der Gasdruck der Versorgung unter einen Schwellwert fällt, die Steuer- oder Regelvorrichtung lediglich mit einer Gasquelle betrieben wird, oder die Blutpumpe oder die Gastauscheinheit vom Normalbetrieb abweichende Betriebszustände aufweist.

Dabei kann auch ein Alarm ausgegeben werden, wenn der Volumenstrom des der Gasaustauscheinheit zugeführten Gases einen Schwellwert unterschreitet. Somit kann der Anwender auf einfache Weise darüber informiert werden, dass der Gasvolumenstrom zu gering ist.

Auch kann ein Anschluss der Steuer- oder Regelvorrichtung an eine zentrale Überwachung im Krankenhaus angekoppelt werden. Dies kann über einen einfachen Anschluss mit auf/ zu Kontakt über den zentralen Patientenhilfsschalter (Nurse call) erfolgen.

Darüber hinaus kann die Steuer- oder Regelvorrichtung eine Überwachungseinrichtung zur Überwachung von Vitalparametern des Patienten aufweisen. Im Ergebnis wird eine Steuer- oder Regelvorrichtung realisiert, die eine Vielzahl von Funktionen bietet.

Die Steuer- oder Regeleinheit kann außerdem zum Steuern oder Regeln einer Bluttemperatur dienen. So kann die Herz-Lungen-Maschine eine Heizung oder Kühlvorrichtung aufweisen, die zum Erwärmen oder Kühlen des vom Patienten entnommenen Blutes dient. Die Steuer- oder Regeleinheit kann die Heizung oder Kühlung derart steuern oder regeln, dass die Bluttemperatur die gewünschte Temperatur aufweist. Das Erwärmen des Blutes kann dann notwendig werden, wenn die mit dem Patienten verbundenen Schläuche sehr lang sind und/oder die Umgebungstemperatur sehr gering ist und daher die Temperatur des in den Schläuchen strömenden Bluts absinkt. Eine Kühlung kann aus therapeutischer Sicht für den Schutz der Organe des Patienten angestrebt werden. Die Kühlung kann durch eine chemische Kühlung und/oder durch einen Phasenübergang realisiert werden. Alternativ kann die Kühlung auch auf andere Weise erfolgen.

Bei einer besonderen Ausführung kann die Steuer- oder Regeleinheit eine Gasanschlusseinheit aufweisen, die mit der Gasquelle oder mehreren Gasquellen fluidisch verbunden sein können. Die Gasanschlusseinheit kann mehrere Anschlüsse aufweisen, die jeweils mit jeweils einer Gasquelle fluidisch verbunden sein können. Die Gasanschlusseinheit kann mit der Mischeinrichtung und/oder der weiteren Mischeinrichtung fluidisch verbindbar sein.

Die Steuer- oder Regeleinheit kann eine weitere Schalteinrichtung aufweisen. Mittels der weiteren Schalteinrichtung kann die Gasanschlusseinheit wahlweise mit der Mischeinrichtung oder der weiteren Mischeinrichtung fluidisch verbunden sein. So kann bei einer ersten Schaltstellung der weiteren Schalteinrichtung die Gasanschlusseinheit mit der Mischeinrichtung fluidisch verbunden sein. Insbesondere können wenigstens zwei Gasquellen, wie die erste Quelle und die zweite Quelle, mit der Mischeinrichtung fluidisch verbunden sein. Bei diesem Fall können der Mischeinrichtung Sauerstoff und Luft, insbesondere Druckluft, zugeführt werden. Darüber hinaus kann die weitere Schalteinrichtung derart ausgebildet sein, dass sie eine Schließstellung aufweist, in der weder die Mischeinrichtung noch die weitere Mischeinrichtung mit der Gasanschlusseinheit fluidisch verbunden sind.

Bei einer zweiten Schaltstellung der weiteren Schalteinrichtung kann die Gasanschlusseinheit mit der weiteren Mischeinrichtung fluidisch verbunden sein. Insbesondere kann die weitere Mischeinrichtung mit einer, insbesondere einzigen, Gasquelle fluidisch verbunden sein. Dabei kann in der zweiten Schaltstellung die weitere Mischeinrichtung ausschließlich mit Sauerstoff versorgt werden. Die weitere Schalteinrichtung kann auch derart ausgebildet und ausgeführt sein, dass unabhängig von deren Stellung die andere Einstelleinrichtung immer mit der Gasanschlusseinheit fluidisch verbunden ist. Somit ist sichergestellt, dass der anderen Einstelleinrichtung immer Gas, wie Sauerstoff, zugeführt wird, das zum Ansteuern der Pumpe verwendet wird.

Darüber hinaus kann die weitere Schalteinrichtung derart ausgebildet sein, dass nach einem Ausfall einer Gasquelle automatisch auf eine andere Gasquelle umgeschaltet wird. Dies bietet sich dann an, wenn beispielsweise in der ersten Quelle nicht mehr ausreichend Sauerstoff vorhanden ist und somit eine Versorgung der Gasaustauscheinheit mit Sauerstoff nicht sichergestellt ist. Bei diesem Fall schaltet die Gasanschlusseinheit automatisch auf die dritte Quelle um, die ebenfalls Sauerstoff zur Verfügung stellt. Im Ergebnis kann somit auf einfache Weise sichergestellt werden, dass der weiteren Schalteinrichtung immer ausreichend Gas, wie beispielsweise Sauerstoff, zugeführt wird.

Darüber hinaus kann die Steuer- oder Regeleinheit ein Überdruckventil aufweisen, mittels dem verhindert wird, dass der Blutpumpe ein zu hoher Gasvolumenstrom zugeführt wird. Außerdem kann die Steuer- oder Regeleinheit ein anderes Überdruckventil aufweisen, mittels dem verhindert wird, dass der Gasaustauscheinheit ein zu hoher Gasvolumenstrom zugeführt wird.

Die Steuer- oder Regelvorrichtung weist ein Gehäuse auf, das vorteilhaft einen Handgriff aufweist. Mittels des Handgriffs kann die Steuer- oder Regelvorrichtung auf einfache Weise transportiert werden. Darüber ist kann das Gehäuse derart ausgebildet sein, dass die Steuer- oder Regeleinheit vollständig innerhalb eines Hohlraums des Gehäuses angeordnet ist. Die Einstelleinrichtung, die weitere Einstelleinrichtung und die andere Einstelleinrichtung können an derselben Gehäuseseite angeordnet sein.

Die erfindungsgemäße Herz-Lungen-Maschine umfasst die Blutpumpe, die Gasaustauscheinheit und die Steuer- oder Regelvorrichtung. Die Steuer- oder Regelvorrichtung ist mit der Blutpumpe und der Gasaustauscheinheit fluidisch verbunden. Darüber hinaus ist die Steuer- oder Regelvorrichtung mit mehreren Gasquellen fluidisch verbunden.

In den Figuren ist der Erfindungsgegenstand schematisch dargestellt und wird anhand der Figuren nachfolgend beschrieben, wobei gleiche oder gleichwirkende Elemente zumeist mit denselben Bezugszeichen versehen sind. Dabei zeigen:
- Fig. 1: eine Darstellung einer Herz-Lungen-Maschine mit einer erfindungsgemäßen Steuer- oder Regelvorrichtung gemäß einer ersten Ausführung,
- Fig. 2: eine Darstellung der Herz-Lungen-Maschine mit der erfindungsgemäßen Steuer- oder Regelvorrichtung gemäß der ersten Ausführung, wobei die Steuer- oder Regelvorrichtung in einem ersten Betriebsmodus betrieben wird,
- Fig. 3: eine Darstellung der Herz-Lungen-Maschine mit der erfindungsgemäßen Steuer- oder Regelvorrichtung gemäß der ersten Ausführung, wobei die Steuer- oder Regelvorrichtung in einem zweiten Betriebsmodus betrieben wird,
- Fig. 4: eine Darstellung der Herz-Lungen-Maschine mit der erfindungsgemäßen Steuer- oder Regelvorrichtung gemäß der ersten Ausführung, wobei die Steuer- oder Regelvorrichtung in einem dritten Betriebsmodus betrieben wird,
- Fig. 5: eine Darstellung der weiteren Mischeinrichtung,
- Fig. 6: eine Darstellung der erfindungsgemäßen Steuer- oder Regelvorrichtung gemäß einer zweiten Ausführung,
- Fig. 7: eine Darstellung der Herz-Lungen-Maschine mit der erfindungsgemäßen Steuer- oder Regelvorrichtung gemäß einer dritten Ausführung,
- Fig. 8: eine Darstellung der erfindungsgemäßen Steuer- oder Regelvorrichtung gemäß einer vierten Ausführung,
- Fig. 9: eine Darstellung der erfindungsgemäßen Steuer- oder Regelvorrichtung gemäß einer fünften Ausführung,
- Fig. 10: eine Darstellung der erfindungsgemäßen Steuer- oder Regelvorrichtung gemäß einer sechsten Ausführung,
- Fig. 11: eine Darstellung der erfindungsgemäßen Steuer- oder Regelvorrichtung gemäß einer siebten Ausführung,
- Fig. 12: eine Darstellung der erfindungsgemäßen Steuer- oder Regelvorrichtung gemäß einer achten Ausführung,
- Fig. 13: eine Darstellung der Herz-Lungen-Maschine mit der erfindungsgemäßen Steuer- oder Regelvorrichtung gemäß einer neunten Ausführung,
- Fig. 14: eine Darstellung der Herz-Lungen-Maschine mit der erfindungsgemäßen Steuer- oder Regelvorrichtung gemäß der neunten Ausführung, in der keine fluidische Verbindungen gezeigt sind,
- Fig. 15: eine perspektivische Darstellung der erfindungsgemäßen Steuer- oder Regelvorrichtung gemäß der neunten Ausführung.

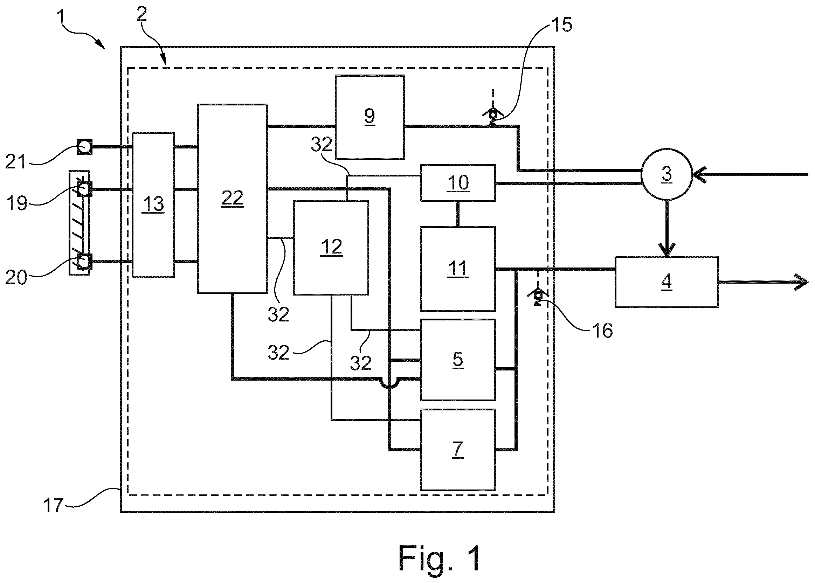

Figur 1 zeigt eine Darstellung einer Herz-Lungen-Maschine mit einer erfindungsgemäßen Steuer- oder Regelvorrichtung 1, einer Blutpumpe 3 und einer Gasaustauscheinheit 4, wie beispielsweise ein Oxygenator. Die Steuer- oder Regelvorrichtung 1 weist eine Steuer- oder Regeleinheit 2 auf, die sowohl einen durch die Blutpumpe 3 geförderten Blutvolumenstrom als auch einen Volumenstrom eines durch die Gasaustauscheinheit 4 strömenden Gases steuert oder regelt. Die Steuer- oder Regeleinheit 2 ist in einem Hohlraum eines Gehäuses 17 der Steuer- oder Regelvorrichtung 1 angeordnet.

Die Steuer-oder Regeleinheit 2 weist eine Gasanschlusseinheit 13 auf, die dazu dient, die Steuer- oder Regelvorrichtung 1 mit mehreren Gasquellen fluidisch zu verbinden. Dazu weist die Gasanschlusseinheit 13 mehrere Anschlüsse auf, die zum Verbinden mit den Gasquellen jeweils mit einer Fluidleitung verbunden sind.

So ist die Gasanschlusseinheit 13 mit einer ersten Quelle 19 fluidisch verbunden. Mittels der ersten Quelle 19 kann der Steuer- oder Regelvorrichtung 1 Sauerstoff zugeführt werden. Darüber hinaus ist die Gasanschlusseinheit 13 mit einer zweiten Quelle 20 fluidisch verbunden. Mittels der zweiten Quelle 20 kann der Steuer- oder Regelvorrichtung 1 Luft, insbesondere Druckluft, zugeführt werden. Außerdem ist die Gasanschlusseinheit 13 mit einer dritten Quelle 21 fluidisch verbunden. Mittels der dritten Quelle 21 kann der Steuer- oder Regelvorrichtung 1 ebenfalls Sauerstoff zugeführt werden. Die erste und zweite Quelle 19, 20 sind beispielsweise in einer Wand eines Krankenzimmers integriert. Die dritte Quelle 21 kann beispielsweise eine Gasflasche sein und als Notreserve dienen, sofern die erste Quelle 19 ausfällt. Es können auch weitere, in den Figuren nicht dargestellte Gasquellen exisitieren, die mit der Steuer- oder Regelvorrichtung 1 verbunden werden können.

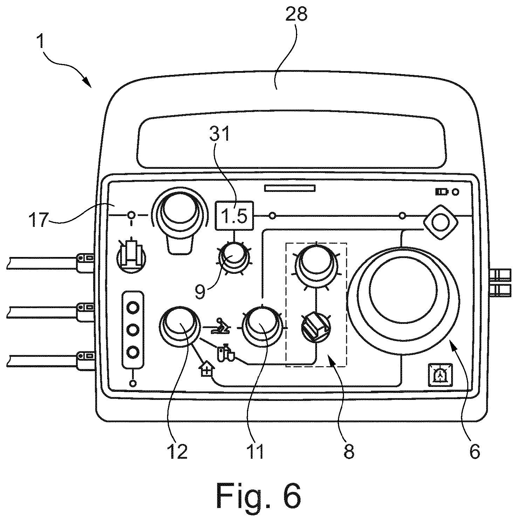

Die Steuer- oder Regeleinheit 2 kann eine Mischeinrichtung 5 aufweisen, die zum Mischen von dem aus der ersten Quelle 19 oder der dritten Quelle 21 stammenden Sauerstoffs und der aus der zweiten Quelle 20 stammenden Druckluft dient. Dabei weist die Steuer- oder Regeleinheit 2 eine in Figur 6 gezeigte Einstelleinrichtung 6 auf, mittels der die Zusammensetzung des aus der Mischeinrichtung 5 strömenden Gases gesteuert oder geregelt werden kann. Darüber hinaus kann mittels der Einstelleinrichtung 6 der Volumenstrom des aus der Mischeinrichtung 5 ausströmenden Gases gesteuert oder geregelt werden, der der stromabwärts angeordneten Gasaustauscheinheit 4 zugeführt wird. Die Einstelleinrichtung 6 weist mehrere Bedienknöpfe auf, mittels deren die Zusammensetzung und der Volumenstrom eingestellt werden können.

Die Steuer- oder Regeleinheit 2 kann außerdem eine weitere Mischeinrichtung 7 aufweisen, die zum Mischen des aus der ersten Quelle 19 oder der dritten Quelle 21 stammenden Sauerstoffs mit Umgebungsluft dient. Dabei weist die Steuer- oder Regeleinheit 2 eine in Figur 6 gezeigte weitere Einstelleinrichtung 8 auf, mittels der die Zusammensetzung des aus der weiteren Mischeinrichtung 7 ausströmenden Gases gesteuert oder geregelt werden kann. Darüber hinaus kann mittels der weiteren Einstelleinrichtung 8 der Volumenstrom des aus der weiteren Mischeinrichtung 7 ausströmenden Gases gesteuert oder geregelt werden, der der stromabwärts angeordneten Gasaustauscheinheit 4 zugeführt wird. Die weitere Einstelleinrichtung 8 weist mehrere Bedienknöpfe auf, mittels deren die Zusammensetzung und der Volumenstrom eingestellt werden können.

Darüber hinaus weist die Steuer- oder Regeleinheit 2 eine andere Einstelleinrichtung 9 auf, die gasstromaufwärts der Blutpumpe 3 angeordnet ist und/oder die stets mit der Gasanschlusseinheit 13 fluidisch verbunden ist. Mittels der anderen Einstelleinrichtung 9 kann der Volumenstrom des zu der Blutpumpe 3 zugeführten Gases gesteuert oder geregelt werden. Dabei kann der Blutpumpe 3 ausschließlich der aus der ersten Quelle 19 oder aus der dritten Quelle 21 strömende Sauerstoff zugeführt werden. Der der Blutpumpe 3 zugeführte Sauerstoff wird zurück in die Steuer- oder Regelvorrichtung 1 geführt. Durch die Zufuhr und Abfuhr von Sauerstoff zu der Blutpumpe 3 wird eine Pumpwirkung erzeugt, die einen Blutvolumenstrom bewirkt. Dabei kann ein Überdruckventil 15 strömungstechnisch zwischen der anderen Einstelleinrichtung 9 und der Blutpumpe 3 angeordnet sein. Durch das Überdruckventil 15 kann verhindert werden, dass der Blutpumpe 3 ein zu hoher Gasvolumenstrom zugeführt wird.

Der Strömungsverlauf des Blutes ist in Figur 1 nur schematisch dargestellt. So strömt das aus dem Patienten entnommene Blut in die Blutpumpe 3 und von dort zur Gasaustauscheinheit 4. In der Gasaustauscheinheit 4 wird das Blut mit Sauerstoff angereichert und strömt wieder zum Patienten zurück. Der Strömungsverlauf des Blutes ist in Figur 1 durch drei Pfeile dargestellt.

Die Steuer- oder Regeleinheit 2 weist eine Schalteinrichtung 10 auf, die gasstromabwärts der Blutpumpe 3 und gasstromaufwärts der Gasaustauscheinheit 4 angeordnet ist. Bei einer Schaltstellung der Schalteinrichtung 10 ist die Blutpumpe 3 mit der Gasaustauscheinheit 4 fluidisch verbunden. Bei einer anderen Schaltstellung der Schalteinrichtung 10 kann der von der Blutpumpe 3 stammende Gasvolumenstrom vollständig an die Umgebung abgegeben werden.

Eine Schalteinstelleinrichtung 11 ist strömungstechnisch zwischen der Schalteinrichtung 10 und der Gasaustauscheinheit 4 angeordnet. Mittels der Schalteinstelleinrichtung 11 kann der der Gasaustauscheinheit 4 zugeführte Gasvolumenstrom gesteuert oder geregelt werden. Die Schalteinstelleinrichtung 11 ist derart ausgebildet, dass der Teil des aus der Blutpumpe 3 strömenden Gasvolumenstroms, der der Gasaustauscheinheit 4 nicht zugeführt werden soll, an die Umgebung abgegeben wird.

Gasstromaufwärts der Gasaustauscheinheit 4 ist ein anderes Überdruckventil 16 angeordnet. Mittels des anderen Überdruckventils 16 kann verhindert werden, dass der Gasaustauscheinheit 4 ein zu großer Gasvolumenstrom zugeführt wird. Der in die Gasaustauscheinheit 4 zugeführte Gasvolumenstrom wird nach Anreicherung des Bluts mit Sauerstoff an die Umgebung abgegeben.

Die Steuer- oder Regeleinheit 2 weist eine weitere Schalteinrichtung 22 auf. Mittels der weiteren Schalteinrichtung 22 ist die Gasanschlusseinheit 13 wahlweise mit der Mischeinrichtung 5 oder der weiteren Mischeinrichtung 7 fluidisch verbunden. Darüber hinaus ist die Mischeinrichtung 5 derart ausgebildet, dass die Gasanschlusseinheit 13, insbesondere stets, mit der anderen Einstelleinrichtung 9 fluidisch verbunden ist. Insbesondere kann die andere Einstelleinrichtung 9 mit der ersten Quelle 19 oder der dritten Quelle 21 stets fluidisch verbunden sein. Die weitere Mischeinrichtung 7 ist außerdem derart ausgebildet, dass sie bei Ausfall der ersten Quelle 19 automatisch auf die dritte Quelle 21 umschaltet.

Die Steuer- oder Regeleinheit 2 weist außerdem eine Auswahleinrichtung 12 auf, mittels der die nachfolgend näher beschriebenen unterschiedlichen Betriebsmodi eingestellt werden können. Die Auswahleinrichtung 12 ist mit der Schalteinrichtung 10, der weiteren Schalteinrichtung 22, der Mischeinrichtung 5 und der weiteren Mischeinrichtung 7 mittels jeweils einer elektrischen Leitung 32 elektrisch verbunden. Die Stellung der Schalteinrichtung 10 und/oder der weiteren Schalteinrichtung 22 hängt von der Stellung der Auswahleinrichtung 12 ab.

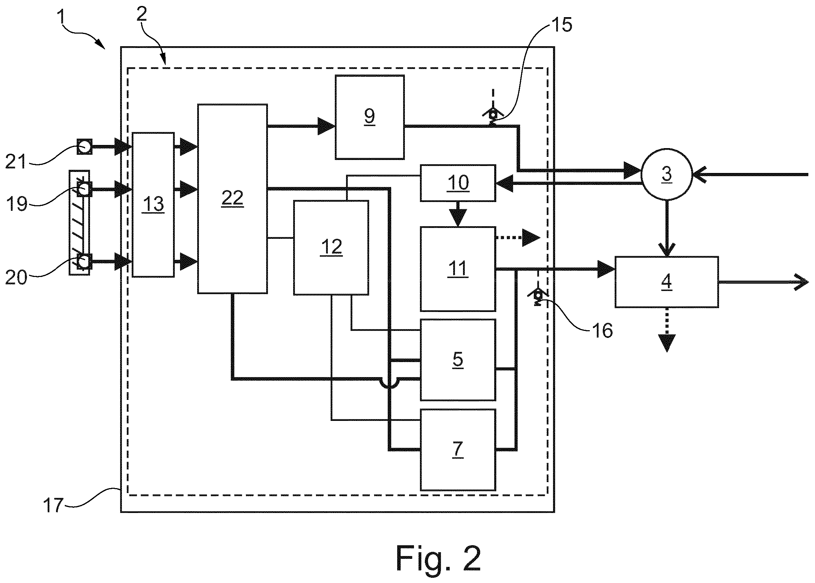

Figur 2 zeigt eine Darstellung der Herz-Lungen-Maschine mit der erfindungsgemäßen Steuer- oder Regelvorrichtung 1 gemäß der ersten Ausführung, wobei die Steuer- oder Regelvorrichtung 1 in einem ersten Betriebsmodus betrieben wird. Der erste Betriebsmodus kann ausgewählt werden, indem der Anwender die Auswahleinrichtung 12 entsprechend betätigt, insbesondere einen Knopf der Auswahleinrichtung 12 in eine entsprechende Stellung dreht.

In Figur 2 sind die Leitungen, durch die ein Gas strömt, mit einem Pfeil dargestellt. So strömt im ersten Betriebsmodus Sauerstoff aus der Gasquelle in die Gasanschlusseinheit 13 und von dort zu der anderen Einstelleinrichtung 9. Ausgehend von der anderen Einstelleinrichtung 9 strömt der Sauerstoff zu der Blutpumpe 3 und von dort zurück in die Steuer- oder Regelvorrichtung 1. Insbesondere strömt der Sauerstoff von der Blutpumpe 3 in die Schalteinrichtung 10. Die Schalteinrichtung 10 befindet sich in der Schaltstellung, so dass das Gas weiter zu der Schalteinstelleinrichtung 11 strömt. Die Schalteinstelleinrichtung 11 ist derart eingestellt, dass ein Teil des aus der Schalteinrichtung 10 strömenden Gases an die Umgebung abgegeben wird, was durch den gestrichelten Pfeil dargestellt ist. Der nicht an die Umgebung abgegebene Teil strömt zur Gasaustauscheinheit 4. Der durch die Gasaustauscheinheit 4 geströmte Teil des Gases wird an die Umgebung abgegeben, was durch den gestrichelten Pfeil dargestellt ist.

Bei dem ersten Betriebsmodus befindet sich die weitere Schalteinrichtung 22 in einer Schließstellung, bei der weder der Mischeinrichtung 5 noch der weiteren Mischeinrichtung 7 Gas zugeführt wird. Darüber hinaus strömt weder von der Mischeinrichtung 5 noch von der weiteren Mischeinrichtung 7 Gas in die Gasaustauscheinheit 7.

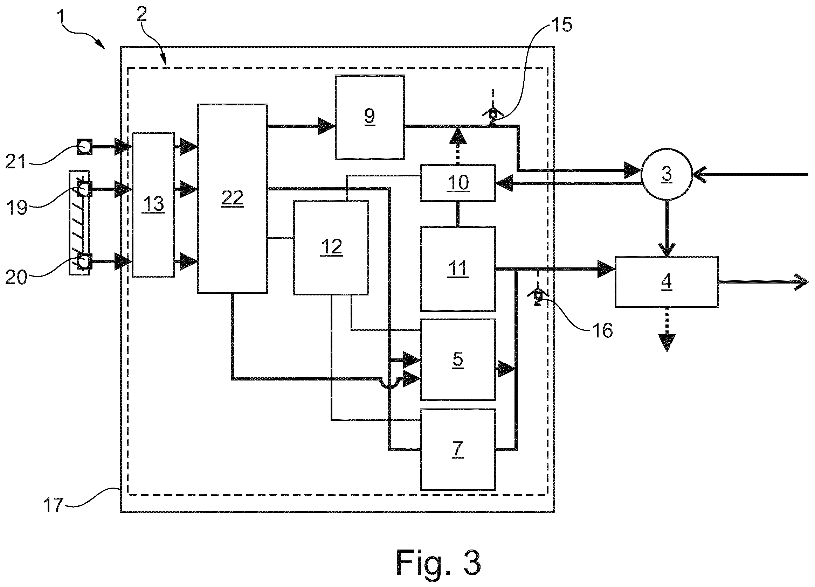

Figur 3 zeigt eine Darstellung der Herz-Lungen-Maschine mit der erfindungsgemäßen Steuer- oder Regelvorrichtung 1 gemäß der ersten Ausführung, wobei die Steuer- oder Regelvorrichtung 1 in einem zweiten Betriebsmodus betrieben wird. Der zweite Betriebsmodus kann ausgewählt werden, indem der Anwender die Auswahleinrichtung 12 entsprechend betätigt, insbesondere einen Knopf der Auswahleinrichtung 12 in eine entsprechende Stellung dreht.

In Figur 3 sind die Leitungen, durch die ein Gas strömt, mit einem Pfeil dargestellt. Bei dem zweiten Betriebsmodus befindet sich die weitere Schalteinrichtung 22 in einer ersten Schaltstellung, bei der die erste oder dritte Quelle 19, 21 und die zweite Quelle 20 mit der Mischvorrichtung 5 fluidisch verbunden sind. Mittels der Einstelleinrichtung 6 wird die Zusammensetzung und/oder der Volumenstrom des aus der Mischeinrichtung 5 ausströmenden Gases gesteuert oder geregelt. Das aus der Mischeinrichtung 5 ausströmende Gas strömt durch die Gasaustauscheinheit 4 und wird anschließend an die Umgebung abgegeben, wie durch den gestrichelten Pfeil dargestellt ist.

Darüber hinaus strömt beim zweiten Betriebsmodus Sauerstoff zur Blutpumpe 3 und von dort zurück zu der Steuer- oder Regelvorrichtung 1. Der Volumenstrom des zu der Blutpumpe 3 strömenden Sauerstoffs kann mittels der anderen Einstelleinrichtung 9 gesteuert oder geregelt werden. Im Gegensatz zu dem ersten Betriebsmodus befindet sich die Schalteinrichtung 10 in der anderen Schaltstellung, bei der die Blutpumpe 3 und die Gasaustauscheinheit 4 mittels der Schalteinrichtung 10 nicht miteinander fluidisch verbunden sind. Dies bedeutet, dass der aus der Blutpumpe 3 strömenden Sauerstoff an die Umgebung abgegeben wird wie durch den gestrichelten Pfeil dargestellt ist.

Bei dem zweiten Betriebsmodus strömt kein Gas durch die weitere Mischeinrichtung 7. Darüber hinaus wird der Gasaustauscheinheit 4 ausschließlich das aus der Mischeinrichtung 5 ausströmende Gas zugeführt.

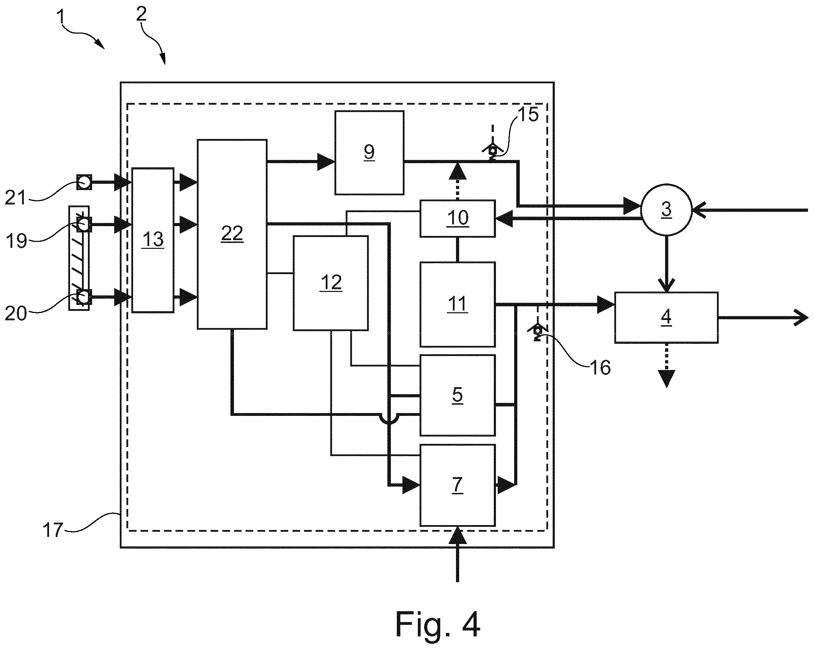

Figur 4 zeigt eine Darstellung der Herz-Lungen-Maschine mit der erfindungsgemäßen Steuer- oder Regelvorrichtung 1 gemäß der ersten Ausführung, wobei die Steuer- oder Regelvorrichtung 1 in einem dritten Betriebsmodus betrieben wird. Der dritte Betriebsmodus kann ausgewählt werden, indem der Anwender die Auswahleinrichtung 12 entsprechend betätigt, insbesondere einen Knopf der Auswahleinrichtung 12 in eine entsprechende Stellung dreht.

In Figur 4 sind die Leitungen, durch die ein Gas strömt, mit einem Pfeil dargestellt. Bei dem dritten Betriebsmodus befindet sich die weitere Schalteinrichtung 22 in der zweiten Schaltstellung, bei der die weitere Mischeinrichtung 7 mit der ersten Quelle 19 fluidisch verbunden ist. Darüber hinaus wird der weiteren Mischeinrichtung 7 Umgebungsluft zugeführt, wie durch den Pfeil dargestellt ist. Das aus der weiteren Mischeinrichtung 7 ausströmende Gas wird der Gasaustauscheinheit 4 zugeführt.

Im Übrigen bestehen keine Unterschiede zu dem zweiten Betriebsmodus. So befindet sich die Schalteinrichtung 10 in der anderen Schaltstellung, so dass der aus der Blutpumpe 3 strömende Sauerstoff an die Umgebung abgegeben wird.

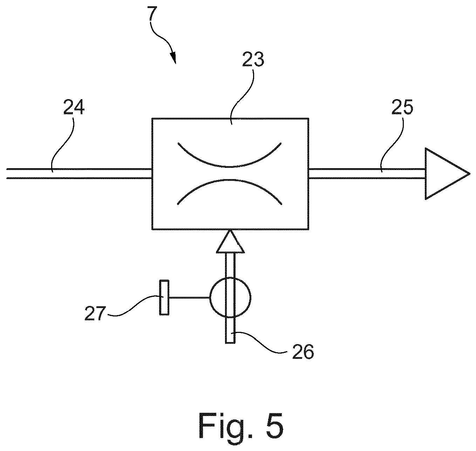

Figur 5 zeigt eine Darstellung der weiteren Mischeinrichtung 7. Aus Figur 5 ist ersichtlich, dass die weitere Mischeinrichtung 7 ein Venturielement 23 aufweist. Das Venturielement 23 ist mittels einer ersten Leitung 24 mit der ersten Quelle 19 oder der dritten Quelle 21 fluidisch verbunden. Darüber hinaus ist das Venturielement 23 mittels einer zweiten Leitung 25 mit der Gasaustauscheinheit 4 fluidisch verbunden. Außerdem ist das Venturielement 23 mittels einer dritten Leitung 26 mit der Umgebung fluidisch verbindbar.

Die weitere Mischeinrichtung 7 weist ein Ventil 27 auf, mittels dem eingestellt werden kann, ob dem Venturielement 23 Umgebungsluft durch die dritte Leitung 26 zugeführt wird.

Fig. 6 zeigt eine Darstellung der erfindungsgemäßen Steuer- oder Regelvorrichtung 1 gemäß einer zweiten Ausführung. Die Ausführung unterscheidet sich von der in Figur 1 gezeigten Ausführung dadurch, dass die Steuer- oder Regelvorrichtung 1 einen Handgriff 28 aufweist, der einstückig mit dem Gehäuse 17 verbunden ist. Die Steuer- oder Regelvorrichtung 1 weist eine Anzeige 31 auf, mittels der beispielsweise Gasvolumenstromwerte angezeigt werden können.

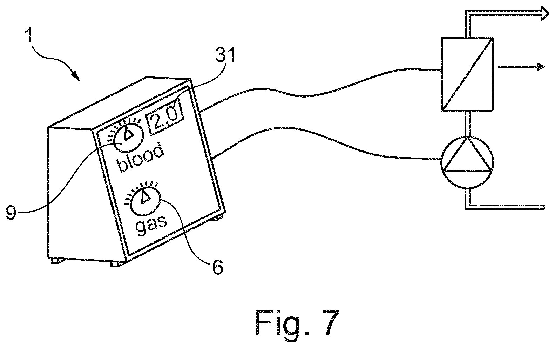

Figur 7 zeigt eine Darstellung der Herz-Lungen-Maschine mit der erfindungsgemäßen Steuer- oder Regelvorrichtung 1 gemäß einer dritten Ausführung. Die Steuer- oder Regelvorrichtung 1 unterscheidet sich von der in Figur 1 dargestellten Steuer- oder Regelvorrichtung 1 darin, dass die Steuer- oder Regelvorrichtung 1 lediglich die Einstelleinrichtung 6 zum Steuern oder Regeln des Volumenstroms des der Gasaustauscheinheit 4 zugeführten Gases und die andere Einstelleinrichtung 9 aufweist, mittels der der Volumenstrom des der Blutpumpe 3 zugeführten Gases steuerbar ist. So weist die Steuer- oder Regelvorrichtung 1 keine weitere Einstelleinrichtung 7 auf.

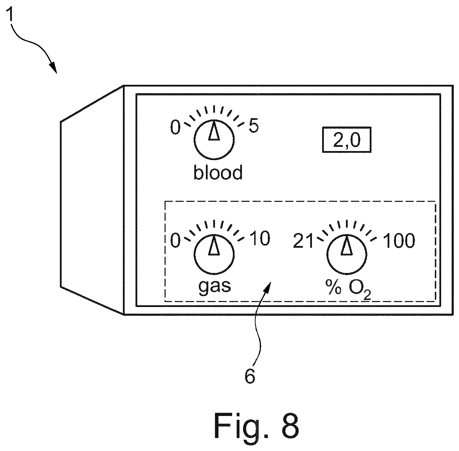

Fig. 8 zeigt eine Darstellung der erfindungsgemäßen Steuer- oder Regelvorrichtung 1 gemäß einer vierten Ausführung. Sie unterscheidet sich von der in Figur 7 dargestellten Ausführung dadurch, dass mittels der Einstelleinrichtung 6 auch die Zusammensetzung des aus der Mischeinrichtung 5 ausströmenden Gases einstellbar ist. Insbesondere kann die Sauerstoffkonzentration des aus der Mischeinrichtung 5 ausströmenden Gases eingestellt werden.

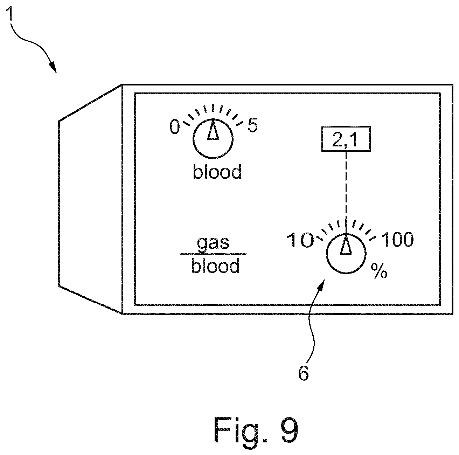

Fig. 9 zeigt eine Darstellung der erfindungsgemäßen Steuer- oder Regelvorrichtung 1 gemäß einer fünften Ausführung. Sie unterscheidet sich von der in Figur 7 dargestellten Ausführung dadurch, dass mittels der Einstelleinrichtung 6 ein Verhältnis zwischen dem Gasvolumenstrom und dem Blutvolumenstrom einstellbar ist. Abhängig von dem eingestellten Verhältnis wird der durch die Mischeinrichtung 5 strömende Gasvolumenstrom und/oder der der Blutpumpe 3 zugeführte Gasvolumenstrom gesteuert oder geregelt, um das gewünschte Verhältnis zu erreichen.

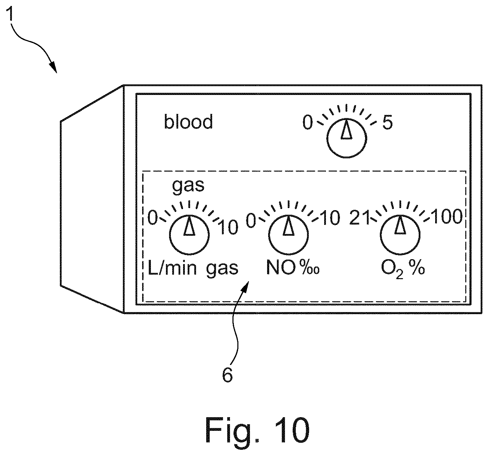

Fig. 10 zeigt eine Darstellung der erfindungsgemäßen Steuer- oder Regelvorrichtung 1 gemäß einer sechsten Ausführung. Diese Ausführung unterscheidet sich von der in Figur 8 gezeigten Ausführung, dass mittels der Einstelleinrichtung 6 auch die Konzentration von Stickstoffmonoxid in dem aus der Mischeinrichtung 5 ausströmenden Gas eingestellt werden kann. Bei dieser Ausführung ist die Mischeinrichtung 5 zusätzlich mit einer vierten Quelle fluidisch verbunden, die Stickstoffmonoxid zur Verfügung stellt.

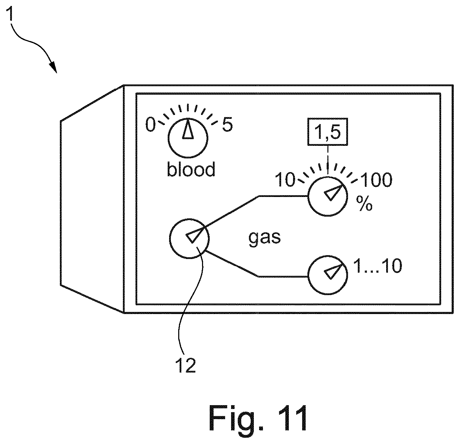

Fig. 11 zeigt eine Darstellung der erfindungsgemäßen Steuer- oder Regelvorrichtung 1 gemäß einer siebten Ausführung. Die Ausführung unterscheidet sich von der in Figur 1 dargestellten Ausführung darin, dass mittels der Steuer- oder Regelvorrichtung 1 lediglich zwei Betriebsmodi mittels der Auswahleinrichtung 12 realisierbar sind.

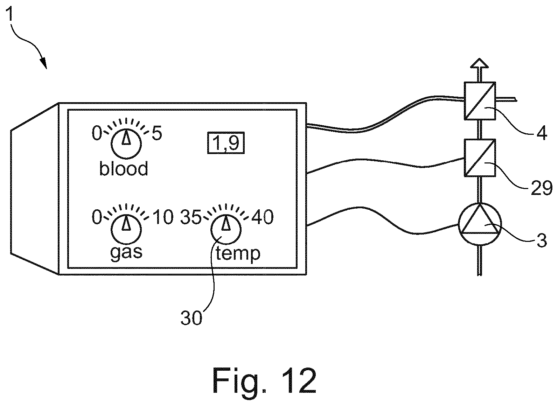

Fig. 12 zeigt eine Darstellung der erfindungsgemäßen Steuer- oder Regelvorrichtung 1 gemäß einer achten Ausführung. Die Ausführung unterscheidet sich von den oben beschriebenen Ausführungen darin, dass die Steuer- oder Regeleinheit 2 zusätzlich dazu dient, eine Heizung 29 der Herz-Lungen-Maschine zu steuern oder regeln. Die Heizung 29 dient dazu, dass aus dem Patienten entnommen Blut aufzuwärmen.

Die Heizung 29 ist strömungstechnisch zwischen der Blutpumpe 3 und der Gasaustauscheinheit 4 angeordnet. Die Steuer- oder Regeleinheit 2 weist ein Einstellelement 30 auf, mittels dem der Anwender die gewünschte Bluttemperatur steuern oder regeln kann.

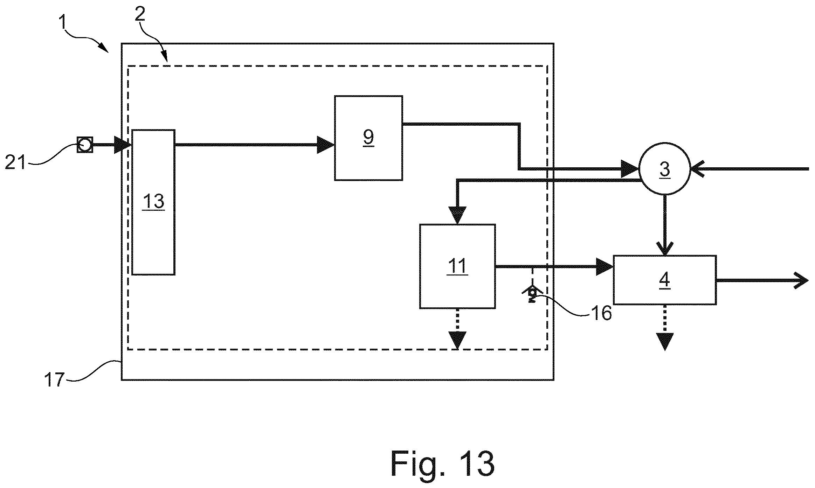

Figur 13 zeigt eine Darstellung der Herz-Lungen-Maschine mit der erfindungsgemäßen Steuer- oder Regelvorrichtung 1 gemäß einer neunten Ausführung. Die in Figur 13 gezeigte Ausführung unterscheidet sich von der in Figur 1 gezeigten Ausführung, dass sie einfacher aufgebaut ist als die in Figur 1 gezeigte Ausführung. So weist die Steuer-oder Regelvorrichtung 1 die andere Einstelleinrichtung 9 und die andere Schalteinrichtung 11 auf.

Die andere Einstelleinrichtung 9 ist mittels der Gasanschlusseinheit 13 mit der dritten Quelle 21 und der Blutpumpe 3 fluidisch verbunden. Insbesondere kann mittels der anderen Einstelleinrichtung 9 der der Blutpumpe 3 zugeführte Gasvolumenstrom und somit der durch die Blutpumpe 3 geförderte Blutvolumenstrom eingestellt werden. Das zugeführte Gas kann Sauerstoff sein.

Die Schalteinrichtung 11 ist mit der Blutpumpe 3 und der Gasaustauscheinheit 4 fluidisch verbunden. Dabei ist die Schalteinrichtung 11 der Blutpumpe 3 strömungstechnisch nachgeschaltet. Die Schalteinrichtung 11 ist derart ausgebildet, dass ein Teil des von der Blutpumpe kommenden Gases an die Umgebung abgegeben werden kann. Das restliche Gas wird zu der Gasaustauscheinheit 4 zugeführt und anschließend an die Umgebung abgegeben.

Das an die Umgebung abgegebene Gas ist jeweils durch einen gestrichelten Pfeil dargestellt.

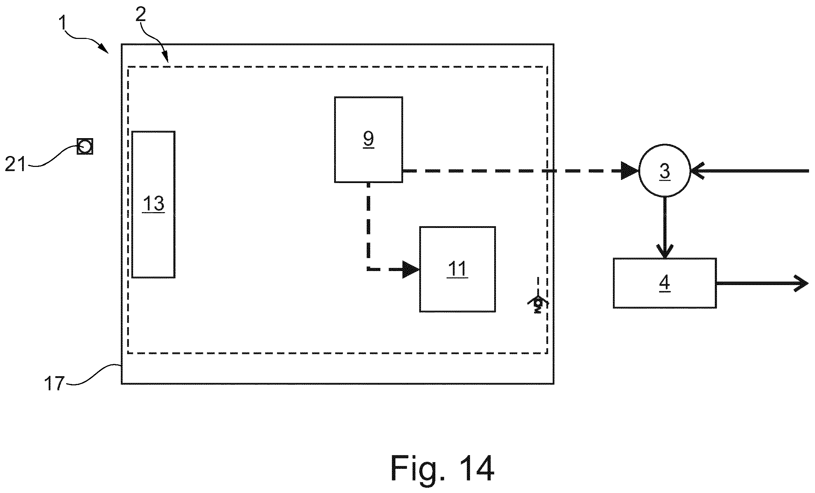

Figur 14 zeigt die Steuer- oder Regelvorrichtung 1 gemäß der neunten Ausführung, bei der die fluidischen Verbindungen nicht eingezeichnet sind. Der Benutzer der Steuer- oder Regelvorrichtung 1 kann durch Betätigen der anderen Einstelleinrichtung 9 den Blutvolumenstrom einstellen, was durch gestrichelten Pfeil dargestellt ist. Konkreter kann durch die andere Einstelleinrichtung 9 der der Blutpumpe 3 zugeführte Gasvolumenstrom eingestellt werden, durch den wiederum der Blutvolumenstrom eingestellt wird. Die Stellung der anderen Einstelleinrichtung 9 wird an die andere Schalteinrichtung 11 übermittelt, was durch den gestrichelten Pfeil dargestellt ist.

Die Steuer- oder Regelvorrichtung 1 steuert oder regelt durch die andere Schalteinrichtung 11 den Gasfluss durch den Gastauscher 4. Dazu kann der Benutzer der Steuer- oder Regelvorrichtung 11 durch Betätigen der anderen Schalteinrichtung 11 den Gasfluss einstellen. Insbesondere können mehrere Regelkurven zum Gasfluss hinterlegt sein. Durch Betätigen der anderen Schalteinrichtung 11 kann der Benutzer eine gewünschte Regelkurve und damit den Gasfluss durch die Gasaustauscheinheit 4 einstellen.

Im Ergebnis wird bei dieser Ausführung durch den Benutzer der Blutvolumenstrom durch Betätigen der anderen Einstelleinrichtung 9 vorgegeben. In Abhängigkeit von dem Blutvolumenstrom steuert oder regelt die Steuer- oder Regelvorrichtung 1, insbesondere über die andere Schalteinrichtung 11, den Gasfluss durch die Gasaustauscheinheit 4.

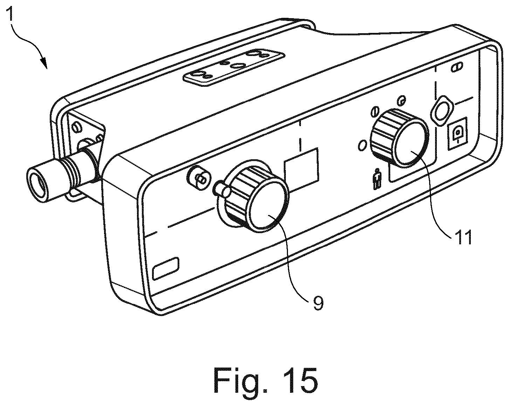

Figur 15 zeigt eine perspektivische Ansicht der Steuer- oder Regelvorrichtung 11 nach der neunten Ausführung. Dabei zeigt Figur 15, dass die andere Einstelleinrichtung 9 und die andere Schalteinrichtung 11 jeweils mit Bedienknopf ausgeführt sind.

### Bezugszeichenliste:

- 1: Steuer- oder Regelvorrichtung
- 2: Steuer- oder Regeleinheit
- 3: Blutpumpe
- 4: Gasaustauscheinheit
- 5: Mischeinrichtung
- 6: Einstelleinrichtung
- 7: weitere Mischeinrichtung
- 8: weitere Einstelleinrichtung
- 9: andere Einstelleinrichtung
- 10: Schalteinrichtung
- 11: Schalteinstelleinrichtung
- 12: Auswahleinrichtung
- 13: Gasanschlusseinheit
- 15: Überdruckventil
- 16: anderes Überdruckventil
- 17: Gehäuse
- 19: erste Quelle
- 20: zweite Quelle
- 21: dritte Quelle
- 22: weitere Schalteinrichtung
- 23: Venturielement
- 24: erste Leitung
- 25: zweite Leitung
- 26: dritte Leitung
- 27: Ventil
- 28: Handgriff
- 29: Heizung
- 30: Einstellelement
- 31: Anzeige
- 32: elektrische Leitung

## Patentansprüche

1. Herz-Lungen-Maschine mit einer Blutpumpe (3), einer Gasaustauscheinheit (4) und einer Steuer- oder Regelvorrichtung (1) mit einer Steuer- oder Regeleinheit (2), die sowohl einen durch eine Blutpumpe (3) förderbaren Blutvolumenstrom als auch einen Volumenstrom eines durch eine Gasaustauscheinheit (4) strömbaren Gases steuert oder regelt, wobei die Steuer- oder Regelvorrichtung (1) ein Gehäuse (17) aufweist, wobei die Steuer- oder Regeleinheit vollständig innerhalb eines Hohlraums des Gehäuses (17) angeordnet ist, wobei die Steuer- oder Regeleinheit (2) eine andere Einstelleinrichtung (9) aufweist, mittels der ein Volumenstrom des zur Blutpumpe (3) zuführbaren Gases steuerbar oder regelbar ist, wobei die Blutpumpe (3) durch das Gas derart angesteuert wird, dass eine Pumpwirkung bewirkt und somit der Blutvolumenstrom gefördert wird, wodurch mittels der anderen Einstelleinrichtung (9) der Blutvolumenstrom gesteuert oder geregelt wird, **dadurch gekennzeichnet, dass** die Steuer- oder Regeleinheit (2) eine Schalteinrichtung (10) aufweist, die mit der Blutpumpe (3) fluidisch verbunden und mit der Gasaustauscheinheit (4) fluidisch verbindbar ist, und dass mittels der Schalteinrichtung (10) eingestellt werden kann, ob das aus der Blutpumpe (3) strömende Gas wahlweise der Gasaustauscheinheit (4) zugeführt wird oder an die Umgebung abgegeben wird.

2. Herz-Lungen-Maschine nach Anspruch 1, **dadurch gekennzeichnet, dass** die Steuer- oder Regeleinheit (2) den Gasvolumenstrom abhängig von dem Blutvolumenstrom steuert oder regelt.

3. Herz-Lungen-Maschine nach einem der Ansprüche 1 bis 2, **dadurch gekennzeichnet, dass** wenigstens ein Algorithmus hinterlegt ist, der das Verhältnis von Gasvolumenstrom und Blutvolumenstrom steuert oder regelt.

4. Herz-Lungen-Maschine nach einem der Ansprüche 1 bis 3, **dadurch gekennzeichnet, dass** die Steuer- oder Regeleinheit (2) eine Mischeinrichtung (5) aufweist, in der wenigstens zwei Gase miteinander mischbar sind.

5. Herz-Lungen-Maschine nach Anspruch 4, **dadurch gekennzeichnet, dass** die Steuer- oder Regeleinheit (2) eine Einstelleinrichtung (6) aufweist, mittels der ein Volumenstrom und/oder eine Zusammensetzung des aus der Mischeinrichtung (5) strömenden Gases steuerbar oder regelbar ist.

6. Herz-Lungen-Maschine nach einem der Ansprüche 4 bis 5, **dadurch gekennzeichnet, dass** die Steuer- oder Regeleinheit (2) eine weitere Mischeinrichtung (7) zum Vermischen von Gas und Umgebungsluft aufweist.

7. Herz-Lungen-Maschine nach einem der Ansprüche 1 bis 6, **dadurch gekennzeichnet, dass** die Steuer- oder Regeleinheit (2) eine Schalteinstelleinrichtung (11) zum Steuern oder Regeln eines Volumenstroms des von der Schalteinrichtung (10) zu der Gasaustauscheinheit (4) zuführbaren Gases aufweist.

8. Herz-Lungen-Maschine nach einem der Ansprüche 1 bis 7, **dadurch gekennzeichnet, dass** die Steuer- oder Regeleinheit (2) eine Auswahleinrichtung (12) aufweist, mittels der wahlweise unterschiedliche Betriebsmodi der Steuer- oder Regelvorrichtung (1) einstellbar sind und/oder, dass mittels der Auswahleinrichtung (12) wahlweise
a. ein erster Betriebsmodus einstellbar ist, bei dem sich die Schalteinrichtung (10) in der Schaltstellung befindet, so dass das dasselbe Gas sowohl der Blutpumpe (3) als auch der Gasaustauscheinheit (4) zuführbar ist oder
b. ein zweiter Betriebsmodus einstellbar ist, bei dem sich die Schalteinrichtung (10) in der anderen Schaltstellung befindet und bei dem das aus der Mischeinrichtung (5) strömende Gas der Gasaustauscheinheit (4) zuführbar ist oder
c. ein dritter Betriebsmodus einstellbar ist, bei dem sich die Schalteinrichtung (10) in der anderen Schaltstellung befindet und bei dem das aus der weiteren Mischeinrichtung (7) strömende Gas der Gasaustauscheinheit (4) zuführbar ist.

9. Herz-Lungen-Maschine nach einem der Ansprüche 1 bis 8, **dadurch gekennzeichnet, dass** die Steuer- und Regelvorrichtung (1) eine Sicherheitseinrichtung zum Überwachen eines Betriebs der Steuer- oder Regelvorrichtung aufweist.

10. Herz-Lungen-Maschine nach einem der Ansprüche 1 bis 9, **dadurch gekennzeichnet, dass** die Steuer- oder Regeleinheit (2) eine Überwachungseinrichtung zur Überwachung von Vitalparametern des Patienten aufweist.

11. Herz-Lungen-Maschine nach einem der Ansprüche 1 bis 10, **dadurch gekennzeichnet, dass** die Steuer- oder Regeleinheit (2) eine Gasanschlusseinheit (13) aufweist, die mit einer Gasquelle oder mehreren Gasquellen fluidisch verbindbar ist.

12. Herz-Lungen-Maschine nach Anspruch 11, **dadurch gekennzeichnet, dass** die Steuer- oder Regeleinheit (2) eine weitere Schalteinrichtung (22) aufweist, die derart ausgebildet ist, dass sie nach einem Ausfall einer Gasquelle automatisch auf eine andere Gasquelle umschaltet.

13. Herz-Lungen-Maschine nach einem der Ansprüche 1 bis 12, **dadurch gekennzeichnet, dass** die Steuer- oder Regeleinheit ein Überdruckventil (15) zum Verhindern, dass der Blutpumpe (3) ein zu hoher Gasvolumenstrom zugeführt wird, aufweist.

14. Herz-Lungen-Maschine nach einem der Ansprüche 1 bis 13, **dadurch gekennzeichnet, dass** die Steuer- oder Regeleinheit ein anderes Überdruckventil (16) zum Verhindern, dass der Gasaustauscheinheit (4) ein zu hoher Gasvolumenstrom zugeführt wird, aufweist.

15. Herz-Lungen-Maschine nach einem der Ansprüche 1 bis 14, **dadurch gekennzeichnet, dass** das Gehäuse (17) einen Handgriff (28) aufweist.

## Claims

1. Heart-lung machine with a blood pump (3), a gas exchange unit (4) and a control or regulation device (1) having a control or regulation unit (2) which controls or regulates both a blood volume flow conveyable through a blood pump (3) and a volume flow of a gas flowable through a gas exchange unit (4), the control or regulation device (1) having a housing (17), the control or regulation unit being arranged completely within a cavity of the housing (17),
wherein the control or regulation unit (2) has another adjusting equipment (9), by means of which a volume flow of the gas that can be supplied to the blood pump (3) can be controlled or regulated,
wherein the blood pump (3) is actuated by the gas in such a way that a pumping effect is caused and thus the blood volume flow is conveyed,
whereby the blood volume flow is controlled or regulated by means of the other adjusting equipment (9),
**characterised in that** the control or regulation unit (2) has a switching equipment (10) which is fluidically connected to the blood pump (3) and can be fluidically connected to the gas exchange unit (4),
and that the switching equipment (10) can be used to adjust whether the gas flowing from the blood pump (3) is optionally supplied to the gas exchange unit (4) or is discharged to the environment.

2. Heart-lung machine according to claim 1, **characterised in that** the control or regulation unit (2) controls or regulates the gas volume flow depending on the blood volume flow.

3. Heart-lung machine according to any of claims 1 to 2, **characterised in that** at least one algorithm is stored that controls or regulates the ratio of gas volume flow and blood volume flow.

4. Heart-lung machine according to any of claims 1 to 3, **characterised in that** the control or regulation unit (2) has a mixing equipment (5) in which at least two gases can be mixed with one another.

5. Heart-lung machine according to claim 4, **characterised in that** the control or regulation unit (2) has an adjusting equipment (6), by means of which a volume flow and/or a composition of the gas flowing from the mixing equipment (5) can be controlled or regulated.

6. Heart-lung machine according to any of claims 4 to 5, **characterised in that** the control or regulation unit (2) has a further mixing equipment (7) for mixing gas and ambient air.

7. Heart-lung machine according to any of claims 1 to 6, **characterised in that** the control or regulation unit (2) has a switching adjustment equipment (11) for controlling or regulating a volume flow of the gas which can be supplied from the switching equipment (10) to the gas exchange unit (4).

8. Heart-lung machine according to any of claims 1 to 7, **characterised in that** the control or regulation unit (2) has a selection equipment (12), by means of which different operating modes of the control or regulation device (1) can optionally be adjusted and/or **in that**, by means of the selection equipment (12), optionally
a. a first operating mode can be adjusted in which the switching equipment (10) is in the switching position, so that the same gas can be supplied to both the blood pump (3) and the gas exchange unit (4) or
b. a second operating mode can be adjusted in which the switching equipment (10) is in the other switching position and in which the gas flowing from the mixing equipment (5) can be supplied to the gas exchange unit (4) or
c. a third operating mode can be adjusted in which the switching equipment (10) is in the other switching position and in which the gas flowing from the further mixing equipment (7) can be supplied to the gas exchange unit (4).

9. Heart-lung machine according to any of claims 1 to 8, **characterised in that** the control and regulation device (1) has a safety equipment for monitoring operation of the control or regulation device.

10. Heart-lung machine according to any of claims 1 to 9, **characterised in that** the control or regulation unit (2) has a monitoring equipment for monitoring vital parameters of the patient.

11. Heart-lung machine according to any of claims 1 to 10, **characterised in that** the control or regulation unit (2) has a gas connection unit (13) which can be fluidically connected to a gas source or a plurality of gas sources.

12. Heart-lung machine according to claim 11, **characterised in that** the control or regulation unit (2) has a further switching equipment (22) which is designed in such a way that it automatically switches to another gas source following a failure of a gas source.

13. Heart-lung machine according to any of claims 1 to 12, **characterised in that** the control or regulation unit has a pressure relief valve (15) for preventing an excessively high gas volume flow from being supplied to the blood pump (3).

14. Heart-lung machine according to any of claims 1 to 13, **characterised in that** the control or regulation unit has another pressure relief valve (16) for preventing an excessively high gas volume flow from being supplied to the gas exchange unit (4).

15. Heart-lung machine according to any of claims 1 to 14, **characterised in that** the housing (17) has a handle (28).

## Revendications

1. Machine cardio-pulmonaire dotée d'une pompe à sang (3), d'une unité d'échange gazeux (4)
et d'un dispositif de commande ou de régulation (1) doté d'une unité de commande ou de régulation (2) qui commande ou régule à la fois un débit volumique de sang pouvant être refoulé par une pompe à sang (3) et un débit volumique de gaz pouvant s'écouler à travers une unité d'échange gazeux (4), ledit dispositif de commande ou de régulation (1) comportant un boîtier (17), ladite unité de commande ou de régulation étant entièrement disposée à l'intérieur d'une cavité du boîtier (17),
ladite unité de commande ou de régulation (2) comportant un autre dispositif de réglage (9) au moyen duquel un débit volumique de gaz pouvant être amené à la pompe à sang (3) peut être commandé ou régulé,
ladite pompe à sang (3) étant commandée par le gaz de manière à produire un effet de pompage et à favoriser ainsi le débit volumique de sang, ce qui permet de commander ou de réguler le débit volumique de sang à l'aide de l'autre dispositif de réglage (9),
**caractérisée en ce que** l'unité de commande ou de régulation (2) comporte un dispositif de commutation (10) qui est relié fluidiquement à la pompe à sang (3) et peut être relié fluidiquement à l'unité d'échange gazeux (4), et que le dispositif de commutation (10) peut être utilisé pour le réglage si le gaz s'écoulant hors de la pompe à sang (3) est sélectivement alimenté vers l'unité d'échange gazeux (4) ou s'il est libéré dans l'environnement.

2. Machine cardio-pulmonaire selon la revendication 1, **caractérisée en ce que** l'unité de commande ou de régulation (2) commande ou régule le débit volumique de gaz en fonction du débit volumique de sang.

3. Machine cardio-pulmonaire selon l'une quelconque des revendications 1 à 2, **caractérisée en ce qu'**au moins un algorithme est enregistré, qui commande ou régule le rapport entre le débit volumique de gaz et le débit volumique de sang.

4. Machine cardio-pulmonaire selon l'une quelconque des revendications 1 à 3, **caractérisée en ce que** l'unité de commande ou de régulation (2) comporte un dispositif de mélange (5) dans lequel au moins deux gaz peuvent être mélangés l'un à l'autre.

5. Machine cardio-pulmonaire selon la revendication 4, **caractérisée en ce que** l'unité de commande ou de régulation (2) comporte un dispositif de réglage (6) au moyen duquel le débit volumique et/ou la composition du gaz s'écoulant du dispositif de mélange (5) peuvent être commandés ou régulés.

6. Machine cardio-pulmonaire selon l'une quelconque des revendications 4 à 5, **caractérisée en ce que** l'unité de commande ou de régulation (2) comporte un dispositif de mélange supplémentaire (7) pour mélanger le gaz et l'air ambiant.

7. Machine cardio-pulmonaire selon l'une quelconque des revendications 1 à 6, **caractérisée en ce que** l'unité de commande ou de régulation (2) comporte un dispositif de réglage de commutation (11) pour commander ou réguler le débit volumique du gaz pouvant être amené du dispositif de commutation (10) à l'unité d'échange gazeux (4).

8. Machine cardio-pulmonaire selon l'une quelconque des revendications 1 à 7, **caractérisée en ce que** l'unité de commande ou de régulation (2) comporte un dispositif de sélection (12) au moyen duquel différents modes de fonctionnement du dispositif de commande ou de régulation (1) peuvent être réglés sélectivement et/ou **en ce que** le dispositif de sélection (12) est sélectivement
a. réglé sur un premier mode de fonctionnement, dans lequel le dispositif de commutation (10) se trouve en position de commutation, de sorte que le même gaz puisse être alimenté à la fois dans la pompe à sang (3) et dans l'unité d'échange gazeux (4), ou
b. réglé sur un deuxième mode de fonctionnement, dans lequel le dispositif de commutation (10) se trouve dans l'autre position de commutation et dans lequel le gaz s'écoulant hors du dispositif de mélange (5) peut être alimenté vers l'unité d'échange gazeux (4) ou
c. réglé sur un troisième mode de fonctionnement, dans lequel le dispositif de commutation (10) se trouve dans l'autre position de commutation et dans lequel le gaz s'écoulant hors du dispositif de mélange supplémentaire (7) peut être alimenté vers l'unité d'échange gazeux (4).

9. Machine cardio-pulmonaire selon l'une des revendications 1 à 8, **caractérisée en ce que** le dispositif de commande et de régulation (1) comporte un dispositif de sécurité pour surveiller le fonctionnement du dispositif de commande ou de régulation.

10. Machine cardio-pulmonaire selon l'une quelconque des revendications 1 à 9, **caractérisée en ce que** l'unité de commande ou de régulation (2) comporte un dispositif de surveillance pour surveiller les paramètres vitaux du patient.

11. Machine cardio-pulmonaire selon l'une quelconque des revendications 1 à 10, **caractérisée en ce que** l'unité de commande ou de régulation (2) comporte une unité de raccordement de gaz (13) qui peut être reliée fluidiquement à une ou plusieurs sources de gaz.

12. Machine cardio-pulmonaire selon la revendication 11, **caractérisée en ce que** l'unité de commande ou de régulation (2) comporte un dispositif de commutation supplémentaire (22) qui est conçu de sorte qu'il commute automatiquement sur une autre source de gaz après la défaillance d'une source de gaz.

13. Machine cardio-pulmonaire selon l'une quelconque des revendications 1 à 12, **caractérisée en ce que** l'unité de commande ou de régulation comporte une soupape de surpression (15) pour empêcher qu'un débit volumique de gaz trop élevé ne soit amené à la pompe à sang (3).

14. Machine cardio-pulmonaire selon l'une quelconque des revendications 1 à 13, **caractérisée en ce que** l'unité de commande ou de régulation comporte une autre soupape de surpression (16) pour empêcher qu'un débit volumique de gaz trop élevé ne soit amené à l'unité d'échange gazeux (4).

15. Machine cardio-pulmonaire selon l'une quelconque des revendications 1 à 14, **caractérisée en ce que** le boîtier (17) comporte une poignée (28).
